# EUROPEAN PATENT APPLICATION

(11) **EP 4 253 553 A1**
(43) Date of publication of application: **04.10.2023**
(21) Application number: 21937750.4
(22) Date of filing: 24.12.2021
(51) Int. Cl.: C12P 19/12, C12P 19/04

(54) **YEAST MANNAN-OLIGOSACCHARIDE, PREPARATION METHOD THEREFOR, AND APPLICATION THEREOF AS YEAST PREBIOTIC**

(30) Priority: 19.04.2021 CN 202110421028
(71) Applicant: Angel Nutritech Co., Ltd., Yichang, Hubei 443003 (CN)
(72) Inventor: XIAO, Minghua, Yichang, Hubei 443003 (CN); CHEN, Zhixian, Yichang, Hubei 443003 (CN); ZHANG, Haibo, Yichang, Hubei 443003 (CN); ZHANG, Yan, Yichang, Hubei 443003 (CN); GONG, Shiyu, Yichang, Hubei 443003 (CN)
(74) Representative: EDP Patent Attorneys B.V.
(86) International application number: PCT/CN2021/141353
(87) International publication number: WO 2022/222528

(57) **Abstract**

The present disclosure provides a yeast mannan-oligosaccharide, a preparation method and a use thereof. The preparation method comprises the following steps: step S1, mixing the yeast cell wall with water to obtain a mixed solution; step S2, adjusting the pH of the mixed solution to 4-9, and carrying out a first enzymolysis with a protease to form a first enzymatic hydrolysate; secondly, subjecting the first enzymatic hydrolysate to a second enzymolysis with a polysaccharidase to obtain a second enzymatic hydrolysate, wherein the usage of the protease is greater than or equal to 0.2% by weight of the yeast cell wall, and the usage of the polysaccharidase is greater than or equal to 0.1% by weight of the yeast cell wall; and step S3, subjecting the second enzymatic hydrolysate to centrifugal separation so as to obtain a supernatant and a centrifugal heavy phase, filtering the supernatant with an ultrafiltration membrane, the retentate being the yeast mannan-oligosaccharide, the permeate being the yeast extract, and the centrifugal heavy phase being the yeast glucan. The yeast mannan-oligosaccharide obtained according to the present disclosure has higher purity, higher yield, the mannan-oligosaccharide is cleaner, which can be fermented by beneficial bacteria, and can be used as prebiotics in foods.

## Description

### Technical Field

The present disclosure relates to the technical field of yeast extraction, in particular to a yeast mannan-oligosaccharide, a preparation method and a use thereof.

### Background

Prebiotics was proposed by G. R. Gibsion et al., and it refers to "indigestible food ingredients, which can selectively stimulate the growth or activity of one or a few bacteria in the colon to have beneficial effects on a host, so as to improve the health of the host". Such activated bacteria should be inherently beneficial, such as bifidobacterium and lactobacillus.

At present, the commonly used prebiotics include oligosaccharides, including fructooligosaccharides, galactooligosaccharides, xylooligosaccharides, isomaltooligosaccharides, soybean oligosaccharides, and inulin, etc. Some microalgae can also be used as prebiotics, such as spirulina, and arthrospira, etc. In addition, polysaccharides (such as Coriolus versicolor polysaccharide, and Daucus Carota nitrogen-containing polysaccharide), protein hydrolysates (such as casein hydrolysates, alpha whey protein, and lactoferrin, etc.), as well as vegetables, Chinese Herbs, and wild plants, etc., in natural plants can also be used as prebiotics.

The yeast cell wall is rich in polysaccharide components, such as yeast beta glucan and mannan-oligosaccharide. Mannan-oligosaccharide is located in the outermost layer of cell wall, with a molecular weight of about 20-200 KD. It has been found that it has a variety of biological activities, which can enhance animal immunity, regulate glucolipid metabolism and maintain intestinal health, and has the effects of promoting growth and anti-oxidation, and thus acts as potential prebiotics. However, at present, it is mainly used as a feed additive, the added value of which is relatively low.

At present, the main preparation method of a yeast mannan-oligosaccharide includes a degradation method such as an enzyme degradation method, an oxidative acidification degradation method, an ultrasonic degradation method and an irradiation modification degradation method, as well as a synthesis method such as microwave solid-state synthesis. Due to the cost and great technical difficulty of synthetic method, the current industrial production mostly adopts the degradation method (LlU Shiqi, et al., Physiological Function of Mannan-Oligosaccharides and Their Application in Livestock and Poultry Production). However, even with the degradation method, the purity of the mannan-oligosaccharides obtained is relatively low, generally about 40%.

CN 102051400 B provides a preparation method of a mannose glycoprotein product, comprising the following steps of: regulating pH to 7.0-10.0 and a temperature to 80-135 °C for processing yeast cell walls. regulating the temperature to 30-70 °C and adding an alkali protease to process the yeast cell walls, centrifugally separating and collecting a supernatant; enabling the supernatant to be in a heat-preservation state for 1-20 hours at a low temperature, and centrifuging to remove a precipitate; and separating the obtained supernatant by a film with the shear molecular weight of 200-400 KD, collecting a permeate liquid, and spray drying to obtain the yeast mannose glycoprotein product. However, the purity of mannan obtained according to the method is not sufficiently high, less than 50%, and the mannose glycoprotein obtained according to the method is mainly used to stabilize wine tartar and protein of wine, with limited application areas.

CN 104862355 B provides a method for co-extracting glucan and mannose glycoprotein from yeast cell walls, comprising homogenizing the yeast cell walls under high pressure, and enzymolysing same with an alkali protease, then centrifuging to obtain a heavy phase as glucan, and filtering the light phase with nanofiltration membrane to obtain the mannose glycoprotein. However, the production cost and noise of high-pressure homogenization are very high.

CN 109912700 A also provides a method for co-extracting glucan and mannose glycoprotein from yeast cell walls, comprising taking yeast cells as a raw material, firstly subjectingthe yeast cells to high-temperature deactivation treatment, centrifuging and drying supernatant to obtain a yeast extract with the heavy phase being the cell walls, performing enzymolysis of yeast cell walls through beta-1,6-glucanase, centrifuging to obtain enzymatic hydrolysate supernatant and an enzymolysis precipitant, and subjecting the enzymatic hydrolysate supernatant to water extraction, alcohol precipitation, centrifugal separation and drying to obtain mannose glycoprotein, after recovery of ethyl alcohol, subjecting residual liquid to concentration and spray drying to obtain a yeast extract, and subjecting the enzymolysis precipitant to degreasing, protease treatment and spray drying to obtain the yeast glucan. Although the mannose glycoprotein produced according to the method is high in purity, the yield is low, and a large amount of ethyl alcohol must be used in the production process, resulting in high production risk and high cost. In addition, the production of glucan according to the method needs an organic solvent (petroleum ether) for degreasing, when applied to foods, it may cause potential safety hazards, and petroleum ether is extremely volatile, which is not environmentally friendly and safe.

In view of above, it is necessary to provide a new preparation method of a yeast mannan-oligosaccharide, through which the yeast mannan-oligosaccharide can be improved in purity and is made more suitable for use in foods as yeast prebiotics.

### Summary

The main object of the present disclosure is to provide a yeast mannan-oligosaccharide and a preparation method and a use thereof, so as to solve the problem of low purity or low yield of yeast mannan-oligosaccharide in the prior art.

In order to achieve the above object, according to one aspect of the present disclosure, a preparation method of a yeast mannan-oligosaccharide is provided, which comprises the following steps: step S1, mixing the yeast cell wall with water to obtain a mixed solution; step S2, adjusting the pH of the mixed solution to 4-9, and then carrying out a first enzymolysis with a protease to form a first enzymatic hydrolysate; secondly, subjecting the first enzymatic hydrolysate to a second enzymolysis with a polysaccharidase to obtain a second enzymatic hydrolysate, wherein the usage of the protease is greater than or equal to 0.2% by weight of the yeast cell wall, and the usage of the polysaccharidase is greater than or equal to 0.1% by weight of the yeast cell wall; and step S3, subjecting the second enzymatic hydrolysate to centrifugal separation so as to obtain a supernatant and a centrifugal heavy phase, filtering the supernatant with an ultrafiltration membrane, the retentate being the yeast mannan-oligosaccharide, the permeate being the yeast extract, and the centrifugal heavy phase being the yeast glucan.

Further, the membrane pore size of the ultrafiltration membrane is 10-100 kD.

Further, in the step S2, the usage of the protease is 0.2-2% by weight of the yeast cell wall, and the usage of the polysaccharidase is 0.1-1% by weight of the yeast cell wall.

Further, the temperature of the first enzymolysis is 45-60 °C and the temperature of the second enzymolysis is 45-60 °C.

Further, the duration of the first enzymolysis is 2-8 h, preferably 3-7 h, and more preferably 4-6 h, and the duration of the second enzymolysis is 1-16 h, preferably 2-14 h, and more preferably 4-12 h.

Further, the protease is selected from one or more of an alkali protease, a plant polypeptidase and a complex protease.

Further, the polysaccharidase is selected from one or more of a cellulase, a mannanase, a complex polysaccharidase and a lyticase.

Further, the concentration by weight of the yeast cell wall in the mixed solution is 8-10%.

According to another aspect of the disclosure, a yeast mannan-oligosaccharide is also provided, which is prepared by the above described preparation method of a yeast mannan-oligosaccharide.

According to another aspect of the disclosure, a use of the above described yeast mannan-oligosaccharide as yeast prebiotics is also provided.

The present disclosure provides a preparation method of a yeast mannan-oligosaccharide, comprising after mixing the yeast cell wall and water into a mixed solution, subjecting same to enzymolysis with a protease and a polysaccharidase successively, then subjecting the enzymatic hydrolysate to centrifugal separation, filtering with an ultrafiltration membrane to obtain the yeast mannan-oligosaccharide, the yeast extract and the yeast glucan at the same time. With the above method, the yield of the yeast mannan-oligosaccharide is more than 25%, and the purity is more than 70%. The yield of the yeast glucan is more than 30% and the purity is more than 70%.

On the one hand, three products, i.e. the yeast extract, the mannan-oligosaccharide and the yeast glucan can be obtained at the same time according to the present disclosure, without using an organic solvent, a strong acid and a strong alkali in the whole process, the process is clean and needs no complex production equipment, and is of low production cost. On the other hand, the yeast mannan-oligosaccharide obtained according to the present disclosure has high purity and high yield. Most importantly, the mannan-oligosaccharide prepared according to the present disclosure is relatively clean, and can be fermented by beneficial bacteria to produce short chain fatty acids, which can promote the proliferation of beneficial bacteria in the intestinal tract, inhibit the growth of harmful bacteria, and can be used in food as prebiotics.

### Detailed Description of the Embodiments

It should be noted that the embodiments and features in the embodiments in the present application can be combined with each other without conflict. The present disclosure will be described in detail below in combination with embodiments.

As described in the background, the yeast mannan-oligosaccharide prepared in the existing technology has problems of low purity, insufficient environmentally friendly production process and high costs, etc. In order to solve the above problems, the present disclosure provides a preparation method of a yeast mannan-oligosaccharide, which comprises the following steps: step S1, mixing the yeast cell wall with water to obtain a mixed solution; step S2, adjusting the pH of the mixed solution to 4-9, and then carrying out a first enzymolysis with a protease to form a first enzymatic hydrolysate; secondly, subjecting the first enzymatic hydrolysate to a second enzymolysis with a polysaccharidase to obtain a second enzymatic hydrolysate, wherein the usage of the protease is greater than or equal to 0.2% by weight of the yeast cell wall, and the usage of the polysaccharidase is greater than or equal to 0.1% by weight of the yeast cell wall; and step S3, subjecting the second enzymatic hydrolysate to centrifugal separation so as to obtain a supernatant and a centrifugal heavy phase, filtering the supernatant with an ultrafiltration membrane, the retentate being the yeast mannan-oligosaccharide, the permeate being the yeast extract, and the centrifugal heavy phase being the yeast glucan.

The above described preparation method comprises after mixing the yeast cell wall and water into a mixed solution, subjecting same to enzymolysis with a protease and a polysaccharidase successively, then subjecting the enzymatic hydrolysate to centrifugal separation, filtering with an ultrafiltration membrane to obtain the yeast mannan-oligosaccharide, the yeast extract and the yeast glucan at the same time. With the above method, the yield of the yeast mannan-oligosaccharide is more than 25%, and the purity is more than 70%. The yield of the yeast glucan is more than 30% and the purity is more than 70%.

On the one hand, three products, i.e. the yeast extract, the mannan-oligosaccharide and the yeast glucan can be obtained at the same time according to the present disclosure, without using an organic solvent, a strong acid and a strong alkali in the whole process, the process is clean and needs no complex production equipment, and is of low production cost. On the other hand, the yeast mannan-oligosaccharide obtained according to the present disclosure has high purity and high yield. Most importantly, the mannan-oligosaccharide prepared according to the present disclosure is relatively clean, and can be fermented by beneficial bacteria to produce short chain fatty acids, which include formic acid, acetic acid, propionic acid, isobutyric acid, butyric acid, isovaleric acid and valeric acid. After being rapidly absorbed by the hindgut, the mannan-oligosaccharide not only stores energy but also reduces osmotic pressure, and the short chain fatty acids play an important role in maintaining the normal functions of large intestine and the morphology and functions of colonic epithelial cells. Short chain fatty acids can also promote the absorption of sodium, in terms of which the butyric acid has stronger effect than acetic acid and propionic acid, furthermore the butyric acid can increase the production of lactobacillus, thereby reducing the number of *Escherichia coli.* Therefore, the mannan-oligosaccharide produced according to the present disclosure can promote the proliferation of intestinal beneficial bacteria (such as bifidobacterium, etc.), inhibit the growth of harmful bacteria (such as pathogenic bacteria, etc.), and can be used as prebiotics in foods.

It should be noted that during the above enzymolysis process, adjusting the pH of the mixed solution to 4-9, and then carrying out a first enzymolysis with a protease to form a first enzymatic hydrolysate; secondly, subjecting the first enzymatic hydrolysate to a second enzymolysis with a polysaccharidase to obtain a second enzymatic hydrolysate, wherein the usage of the protease is greater than or equal to 0.2% by weight of the yeast cell wall, and the usage of the polysaccharidase is greater than or equal to 0.1% by weight of the yeast cell wall. This can not only enable the yeast cell wall to complete relatively sufficient proteolysis and polysaccharidase enzymolysis successively to form more yeast extracts, yeast mannan-oligosaccharides and yeast glucans, but also make use of the above pH conditions and enzyme usage to form a molecular weight difference between the yeast extracts and the yeast mannan-oligosaccharides, which is conducive to filtration and separation. Moreover, the yield and purity of the three products can be optimized through the proteolysis followed by the polysaccharidase enzymolysis.

In order to more sufficiently separate the yeast extracts and the mannan-oligosaccharides, in a preferred embodiment, the filtration pore size of the ultrafiltration membrane is 10-100 kD (kilodalton).

Considering further enzymolysis of protein and polysaccharide in cell wall, and avoiding waste of raw materials as much as possible at the same time, and further improving the prebiotics efficacy of mannan-oligosaccharides, in a preferred embodiment, in step S2, the usage of the protease is 0.2-2% by weight of the yeast cell wall, and the usage of the polysaccharidase is 0.1-1 % by weight of the yeast cell wall. More preferably, the temperature of the first enzymolysis is 45-60 °C and the temperature of the second enzymolysis is 45-60 °C. Within the above temperature range, the protease and the polysaccharidase have better activities.

In a preferred embodiment, the duration of the first enzymolysis is 2-8 h, preferably 3-7 h, and more preferably 4-6 h, and the duration of the second enzymolysis is 1-16 h, preferably 2-14 h, and more preferably 4-12 h. Within the above duration range, the enzymolysis is more sufficient.

Preferably, the protease includes but is not limited to one or more of an alkali protease, a plant polypeptidase and a complex protease. Preferably, the polysaccharidase includes but is not limited to one or more of a cellulase, a mannanase, a complex polysaccharidase and a lyticase. Specifically, proteases and polysaccharidases in the following table can be selected.

| Reagents | Purity or Model | Manufacturer |
|---|---|---|
| Beta-glucanase | 18000U/g | Angel Yeast Co., Ltd |
| Alpha-glucanase | 120000 U/g | Novozymes (China) Biotechnology Co.,Ltd. |
| Cellulase | 10000 U/g | Nanning Pangbo Biological Engineering Co., Ltd. |
| lyticase | 100000 U/g | Nanning Pangbo Biological Engineering Co., Ltd. |
| Alpha-mannanase | 25000 U/g | Nanning Pangbo Biological Engineering Co., Ltd. |
| Complex polysaccharidase | Viscozyme | Novozymes (China) Biotechnology Co.,Ltd. |
| Alkali protease | Alcalase 2.4L | Novozymes (China) Biotechnology Co.,Ltd. |
| Neutral protease | Neutrase 0.8L | Novozymes (China) Biotechnology Co.,Ltd. |
| Papain | 600000 U/g | Nanning Pangbo Biological Engineering Co., Ltd. |
| Plant polypeptidase | 600000 U/g | Nanning Pangbo Biological Engineering Co., Ltd. |
| Bromelain | 800000 U/g | Nanning Pangbo Biological Engineering Co., Ltd. |
| Thermoase C | 1000000 U/g | Amano Enzyme (Shanghai) Business Limited company |
| Protin SD NY | 1000000 U/g | Amano Enzyme (Shanghai) Business Limited company |
| Complex protease | 100000 U/g | Angel Yeast Co., Ltd |
| Yeast cell wall | Food grade | Angel Yeast Co., Ltd |
| Sodium hydroxide | 99.9% | Xilong Chemical Co., Ltd. |
| Hydrochloric acid | 99.9% | Xilong Chemical Co., Ltd. |

More preferably, the concentration by weight of the yeast cell wall in the mixed solution is 8-10%.

In the actual operation process, after the filtration via ultrafiltration membrane, the permeate is concentrated and spray dried to obtain a small molecular yeast extract. The retentate liquid is spray dried to obtain the yeast mannan-oligosaccharide product. The centrifugal heavy phase is spray dried to obtain the yeast glucan product.

According to another aspect of the present disclosure, a yeast mannan-oligosaccharide prepared by the above described preparation method is provided. As mentioned above, the yeast mannan-oligosaccharide has high purity and clean ingredients, can promote the proliferation of intestinal beneficial bacteria (such as bifidobacterium, etc.), inhibit the growth of harmful bacteria (such as pathogenic bacteria, etc.), and can be used as prebiotics in foods.

The present disclosure furtehr provides a use of the above described yeast mannan-oligosaccharide as yeast prebiotics.

The present application will be further described in detail below in combination with specific examples, which cannot be understood as limiting the scope of protection claimed in the present application.

### Example 1

10 kg of yeast cell wall was taken, and water was added to a constant volume of 100 kg.

The temperature was raised to 50 °C, pH was adjusted to 9.0, 20 g of an alkali protease (Alcalase 2.4L, Novozymes) was added for enzymolysis 8 h, and then 10 g of mannanase (25000 U/g, Nanning Pangbo) was added for enzymolysis 16 h.

After the completion of enzymolysis, the mixture was centrifuged, the supernatant was filtered with an ultrafiltration membrane (10 KD), and the permeate was concentrated and spray dried to obtain 3 kg small molecular yeast extract. After the retentate liquid was spray dried, 2.5 kg of mannan-oligosaccharide was obtained with a purity of 71%. After the centrifugal heavy phase was spray dried, 3 kg of yeast glucan was obtained with a purity of 70.5%.

### Example 2

10 kg of yeast cell wall was taken, and water was added to a constant volume of 100 kg.

The temperature was raised to 50 °C, pH was adjusted to 4.0, 200 g of plant polypeptidase (soybean polypeptide hydrolase, 600000 U/g, Nanning Pangbo) was added for enzymolysis 2 h, and then 100 g of cellulase (10000 U/g, Nanning Pangbo) was added for enzymolysis1 h.

After the completion of enzymolysis, the mixture was centrifuged, the supernatant was filtered with an ultrafiltration membrane (100 KD), and the permeate was concentrated and spray dried to obtain 2.8 kg small molecular yeast extract. After the retentate liquid was spray dried, 2.6kg of mannan-oligosaccharide was obtained with a purity of 72%. After the centrifugal heavy phase was spray dried, 3.4 kg of yeast glucan was obtained with a purity of 70.3%.

### Example 3

10 kg of yeast cell wall was taken, and water was added to a constant volume of 100 kg.

The temperature was raised to 50 °C, pH was adjusted to 5.0, 40 g of complex protease (100000 U/g, Angel Yeast Co., Ltd.) was added for enzymolysis 2 h, and then 20 g of complex polysaccharidase (Viscozyme, Novozymes) was added for enzymolysis 14 h.

After the completion of enzymolysis, the mixture was centrifuged, the supernatant was filtered with an ultrafiltration membrane (10 KD), and the permeate was concentrated and spray dried to obtain 2.6kg small molecular yeast extract. After the retentate liquid was spray dried, 2.8kg of mannan-oligosaccharide was obtained with a purity of 71%. After the centrifugal heavy phase was spray dried, 3.4 kg of yeast glucan was obtained with a purity of 71%.

### Example 4

10 kg of yeast cell wall was taken, and water was added to a constant volume of 100 kg.

The temperature was raised to 50 °C, pH was adjusted to 8.0, 180 g of an alkali protease (Alcalase 2.4L, Novozymes) was added for enzymolysis 7 h, and then 90 g of lyticase (100000 U/g, Nanning Pangbo) was added for enzymolysis 2 h.

After the completion of enzymolysis, the mixture was centrifuged, the supernatant was filtered with an ultrafiltration membrane (50 KD), and the permeate was concentrated and spray dried to obtain 2.9 kg small molecular yeast extract. After the retentate liquid was spray dried, 2.9kg of mannan-oligosaccharide was obtained with a purity of 70.3%. After the centrifugal heavy phase was spray dried, 3.0 kg of yeast glucan was obtained with a purity of 74%.

### Example 5

10 kg of yeast cell wall was taken, and water was added to a constant volume of 110kg.

The temperature was raised to 50 °C, pH was adjusted to 6.0, 50 g of plant polypeptidase (soybean polypeptide hydrolase, 600000 U/g, Nanning Pangbo) was added for enzymolysis 4 h, and then 50 g of mannanase (25000 U/g, Nanning Pangbo) was added for enzymolysis 4 h.

After the completion of enzymolysis, the mixture was centrifuged, the supernatant was filtered with an ultrafiltration membrane (100 KD), and the permeate was concentrated and spray dried to obtain 3.0 kg small molecular yeast extract. After the retentate liquid was spray dried, 2.6kg of mannan-oligosaccharide was obtained with a purity of 72%. After the centrifugal heavy phase was spray dried, 3.1 kg of yeast glucan was obtained with a purity of 71.5%.

### Example 6

10 kg of yeast cell wall was taken, and water was added to a constant volume of 125kg.

The temperature was raised to 50 °C, pH was adjusted to 7.0, 170 g of complex protease (100000 U/g, Angel Yeast Co., Ltd.) was added for enzymolysis 6 h, and then 60 g of cellulase (10000 U/g, Nanning Pangbo) was added for enzymolysis 2 h.

After the completion of enzymolysis, the mixture was centrifuged, the supernatant was filtered with an ultrafiltration membrane (50 KD), and the permeate was concentrated and spray dried to obtain 2.9 kg small molecular yeast extract. After the retentate liquid was spray dried, 2.6kg of mannan-oligosaccharide was obtained with a purity of 71.5%. After the centrifugal heavy phase was spray dried, 3.2 kg of yeast glucan was obtained with a purity of 70.7%.

### Example 7

1) 10 kg of yeast cell wall was taken, and water was added to a constant volume of 125 kg.
2) The temperature was raised to 50°C, pH was adjusted to 6.0, 210 g of plant polypeptidase (soybean polypeptide hydrolase, 600000 U/g, Nanning Pangbo) was added for enzymolysis 4 h, and then 50 g of mannanase (25000 U/g, Nanning Pangbo) was added for enzymolysis 4 h.
3) After the completion of enzymolysis, the mixture was centrifuged, the supernatant was filtered with an ultrafiltration membrane (100 KD), and the permeate was concentrated and spray dried to obtain 3.0 kg small molecular yeast extract. After the retentate liquid was spray dried, 2.5 kg of mannan-oligosaccharide was obtained with a purity of 70.3%. After the centrifugal heavy phase was spray dried, 3.1 kg of yeast glucan was obtained with a purity of 70.5%.

### Example 8

10 kg of yeast cell wall was taken, and water was added to a constant volume of 110kg.

The temperature was raised to 50 °C, pH was adjusted to 6.0, 50 g of plant polypeptidase (soybean polypeptide hydrolase, 600000 U/g, Nanning Pangbo) was added for enzymolysis 4 h, and then 110g of mannanase (25000 U/g, Nanning Pangbo) was added for enzymolysis 4 h.

After the completion of enzymolysis, the mixture was centrifuged, the supernatant was filtered with an ultrafiltration membrane (100 KD), and the permeate was concentrated and spray dried to obtain 3.0 kg small molecular yeast extract. After the retentate liquid was spray dried, 2.7kg of mannan-oligosaccharide was obtained with a purity of 70.1%. After the centrifugal heavy phase was spray dried, 2.9 kg of yeast glucan was obtained with a purity of 70.3%.

### Example 9

10 kg of yeast cell wall was taken, and water was added to a constant volume of 100 kg.

The temperature was raised to 45 °C, pH was adjusted to 9.0, 20 g of an alkali protease (Alcalase 2.4L, Novozymes) was added for enzymolysis 8 h, and then 10 g of mannanase (25000 U/g, Nanning Pangbo) was added for enzymolysis 16 h.

After the completion of enzymolysis, the mixture was centrifuged, the supernatant was filtered with an ultrafiltration membrane (10 KD), and the permeate was concentrated and spray dried to obtain 2.6kg small molecular yeast extract. After the retentate liquid was spray dried, 3.0kg of mannan-oligosaccharide was obtained with a purity of 70.5%. After the centrifugal heavy phase was spray dried, 3.1kg of yeast glucan was obtained with a purity of 70.5%.

### Example 10

10 kg of yeast cell wall was taken, and water was added to a constant volume of 100 kg.

The temperature was raised to 60 °C, pH was adjusted to 9.0, 20 g of an alkali protease (Alcalase 2.4L, Novozymes) was added for enzymolysis 8 h, and then 10 g of mannanase (25000 U/g, Nanning Pangbo) was added for enzymolysis 16 h.

After the completion of enzymolysis, the mixture was centrifuged, the supernatant was filtered with an ultrafiltration membrane (10 KD), and the permeate was concentrated and spray dried to obtain 2.8kg small molecular yeast extract. After the retentate liquid was spray dried, 2.8kg of mannan-oligosaccharide was obtained with a purity of 70.8%. After the centrifugal heavy phase was spray dried, 3.1kg of yeast glucan was obtained with a purity of 70.6%.

### Example 11

10 kg of yeast cell wall was taken, and water was added to a constant volume of 110kg.

The temperature was raised to 40 °C, pH was adjusted to 6.0, 50 g of plant polypeptidase (soybean polypeptide hydrolase, 600000 U/g, Nanning Pangbo) was added for enzymolysis 4 h, and then 50 g of mannanase (25000 U/g, Nanning Pangbo) was added for enzymolysis 4 h.

After the completion of enzymolysis, the mixture was centrifuged, the supernatant was filtered with an ultrafiltration membrane (100 KD), and the permeate was concentrated and spray dried to obtain 2.2 kg small molecular yeast extract. After the retentate liquid was spray dried, 3.0kg of mannan-oligosaccharide was obtained with a purity of 68.1%. After the centrifugal heavy phase was spray dried, 3.1 kg of yeast glucan was obtained with a purity of 68.5%.

### Example 12

10 kg of yeast cell wall was taken, and water was added to a constant volume of 110kg.

The temperature was raised to 65 °C, pH was adjusted to 6.0, 50 g of plant polypeptidase (soybean polypeptide hydrolase, 600000 U/g, Nanning Pangbo) was added for enzymolysis 4 h, and then 50 g of mannanase (25000 U/g, Nanning Pangbo) was added for enzymolysis 4 h.

After the completion of enzymolysis, the mixture was centrifuged, the supernatant was filtered with an ultrafiltration membrane (100 KD), and the permeate was concentrated and spray dried to obtain 2.3 kg small molecular yeast extract. After the retentate liquid was spray dried, 2.8 kg of mannan-oligosaccharide was obtained with a purity of 68.4 %. After the centrifugal heavy phase was spray dried, 3.2 kg of yeast glucan was obtained with a purity of 67.3%.

### Example 13

10 kg of yeast cell wall was taken, and water was added to a constant volume of 110kg.

The temperature was raised to 50 °C, pH was adjusted to 6.0, 50 g of plant polypeptidase (soybean polypeptide hydrolase, 600000 U/g, Nanning Pangbo) was added for enzymolysis 4 h, and then 50 g of mannanase (25000 U/g, Nanning Pangbo) was added for enzymolysis 4 h.

After the completion of enzymolysis, the mixture was centrifuged, the supernatant was filtered with an ultrafiltration membrane (5 KD), and the permeate was concentrated and spray dried to obtain 2.1 kg small molecular yeast extract. After the retentate liquid was spray dried, 3.5kg of mannan-oligosaccharide was obtained with a purity of 68.5%. After the centrifugal heavy phase was spray dried, 3.1 kg of yeast glucan was obtained with a purity of 70.5%.

### Example 14

10 kg of yeast cell wall was taken, and water was added to a constant volume of 110kg.

The temperature was raised to 50 °C, pH was adjusted to 6.0, 50 g of plant polypeptidase (soybean polypeptide hydrolase, 600000 U/g, Nanning Pangbo) was added for enzymolysis 4 h, and then 50 g of mannanase (25000 U/g, Nanning Pangbo) was added for enzymolysis 4 h.

After the completion of enzymolysis, the mixture was centrifuged, the supernatant was filtered with an ultrafiltration membrane (200 KD), and the permeate was concentrated and spray dried to obtain 3.5 kg small molecular yeast extract. After the retentate liquid was spray dried, 2.2kg of mannan-oligosaccharide was obtained with a purity of 70%. After the centrifugal heavy phase was spray dried, 2.9 kg of yeast glucan was obtained with a purity of 71.5%.

### Comparative example 1 (with Example 5 as reference)

10 kg of yeast cell wall was taken, and water was added to a constant volume of 110kg.

The temperature was raised to 50 °C, pH was adjusted to 3.0, 50 g of plant polypeptidase (soybean polypeptide hydrolase, 600000 U/g, Nanning Pangbo) was added for enzymolysis 4 h, and then 50 g of mannanase (25000 U/g, Nanning Pangbo) was added for enzymolysis 4 h.

After the completion of enzymolysis, the mixture was centrifuged, the supernatant was filtered with an ultrafiltration membrane (100 KD), and the permeate was concentrated and spray dried to obtain 2.1 kg small molecular yeast extract. After the retentate liquid was spray dried, 2.2kg of mannan-oligosaccharide was obtained with a purity of 65 %. After the centrifugal heavy phase was spray dried, 3.9 kg of yeast glucan was obtained with a purity of 66 %.

### Comparative example 2 (with Example 5 as reference)

10 kg of yeast cell wall was taken, and water was added to a constant volume of 110kg.

The temperature was raised to 50 °C, pH was adjusted to 10.0, 50 g of plant polypeptidase (soybean polypeptide hydrolase, 600000 U/g, Nanning Pangbo) was added for enzymolysis 4 h, and then 50 g of mannanase (25000 U/g, Nanning Pangbo) was added for enzymolysis 4 h.

After the completion of enzymolysis, the mixture was centrifuged, the supernatant was filtered with an ultrafiltration membrane (100 KD), and the permeate was concentrated and spray dried to obtain 2.5kg small molecular yeast extract. After the retentate liquid was spray dried, 2.0kg of mannan-oligosaccharide was obtained with a purity of 64%. After the centrifugal heavy phase was spray dried, 3.9 kg of yeast glucan was obtained with a purity of 62%.

### Comparative example 3 (with Example 5 as reference)

10 kg of yeast cell wall was taken, and water was added to a constant volume of 110kg.

The temperature was raised to 50 °C, pH was adjusted to 6.0, 10g of plant polypeptidase (soybean polypeptide hydrolase, 600000 U/g, Nanning Pangbo) was added for enzymolysis 4 h, and then 50 g of mannanase (25000 U/g, Nanning Pangbo) was added for enzymolysis 4 h.

After the completion of enzymolysis, the mixture was centrifuged, the supernatant was filtered with an ultrafiltration membrane (100 KD), and the permeate was concentrated and spray dried to obtain 1.8 kg small molecular yeast extract. After the retentate liquid was spray dried, 2.1kg of mannan-oligosaccharide was obtained with a purity of 70%. After the centrifugal heavy phase was spray dried, 3.8 kg of yeast glucan was obtained with a purity of 63%.

### Comparative example 4 (with Example 5 as reference)

10 kg of yeast cell wall was taken, and water was added to a constant volume of 110kg.

The temperature was raised to 50 °C, pH was adjusted to 6.0, 50 g of plant polypeptidase (soybean polypeptide hydrolase, 600000 U/g, Nanning Pangbo) was added for enzymolysis 4 h, and then 8g of mannanase (25000 U/g, Nanning Pangbo) was added for enzymolysis 4 h.

After the completion of enzymolysis, the mixture was centrifuged, the supernatant was filtered with an ultrafiltration membrane (100 KD), and the permeate was concentrated and spray dried to obtain 2.5 kg small molecular yeast extract. After the retentate liquid was spray dried, 2.1kg of mannan-oligosaccharide was obtained with a purity of 67.3%. After the centrifugal heavy phase was spray dried, 3.5 kg of yeast glucan was obtained with a purity of 65.8%.

The above descriptions are only the preferred examples of the present disclosure, and are not intended to limit thereto. For those skilled in the art, various modifications and changes can be made to the present disclosure. Any modifications, equivalent substitutions, improvements, and the like made within the spirit and principle of the present disclosure shall be included into the protection scope of the present disclosure.

## Claims

1. A preparation method of a yeast mannan-oligosaccharide, comprising the following steps:
step S1, mixing the yeast cell wall with water to obtain a mixed solution;
step S2, adjusting the pH of the mixed solution to 4-9, and then carrying out a first enzymolysis with a protease to form a first enzymatic hydrolysate; secondly, subjecting the first enzymatic hydrolysate to a second enzymolysis with a polysaccharidase to obtain a second enzymatic hydrolysate, wherein the usage of the protease is greater than or equal to 0.2% by weight of the yeast cell wall, and the usage of the polysaccharidase is greater than or equal to 0.1% by weight of the yeast cell wall; and
step S3, subjecting the second enzymatic hydrolysate to centrifugal separation so as to obtain a supernatant and a centrifugal heavy phase, filtering the supernatant with an ultrafiltration membrane, the retentate being the yeast mannan-oligosaccharide, the permeate being the yeast extract, and the centrifugal heavy phase being the yeast glucan.

2. The preparation method of the yeast mannan-oligosaccharide according to claim 1, wherein the membrane pore size of the ultrafiltration membrane is 10-100 kD.

3. The preparation method of the yeast mannan-oligosaccharide according to claim 1, wherein in the step S2, the usage of the protease is 0.2-2% by weight of the yeast cell wall, and the usage of the polysaccharidase is 0.1-1% by weight of the yeast cell wall.

4. The preparation method of the yeast mannan-oligosaccharide according to any one of claims 1-3, wherein the temperature of the first enzymolysis is 45-60 °C and the temperature of the second enzymolysis is 45-60°C.

5. The preparation method of the yeast mannan-oligosaccharide according to claim 4, wherein the duration of the first enzymolysis is 2-8 h, preferably 3-7 h, and more preferably 4-6 h; the duration of the second enzymolysis is 1-16 h, preferably 2-14 h, and more preferably 4-12 h.

6. The preparation method of the yeast mannan-oligosaccharide according to any one of claims 1-5, wherein the protease is selected from one or more of an alkali protease, a plant polypeptidase and a complex protease.

7. The preparation method of the yeast mannan-oligosaccharide according to any one of claims 1-5, wherein the polysaccharidase is selected from one or more of a cellulase, a mannanase, a complex polysaccharidase and a lyticase.

8. The preparation method of the yeast mannan-oligosaccharide according to any one of claims 1-5, wherein the concentration by weight of the yeast cell wall in the mixed solution is 8-10%.

9. A yeast mannan-oligosaccharide, wherein it is prepared by the preparation method of a yeast mannan-oligosaccharide according to any one of claims 1-8.

10. A use of the yeast mannan-oligosaccharide according to claim 9 as yeast prebiotics.
